Europäisches Patentamt

European Patent Office

Office européen des brevets

(11) Publication number: 0 209 720
B1

# EUROPEAN PATENT SPECIFICATION

(45) Date of publication of patent specification: 19.09.90

(21) Application number: 86108204.8

(22) Date of filing: 16.06.86

(51) Int. Cl.⁵: C 07 D 487/08, B 01 J 2/30

(54) Flowable triethylenediamine.

(30) Priority: 21.06.85 US 747621

(43) Date of publication of application:
28.01.87 Bulletin 87/05

(45) Publication of the grant of the patent:
19.09.90 Bulletin 90/38

(84) Designated Contracting States:
BE DE FR GB IT NL SE

(56) References cited:
AU-B- 472 194
FR-A-2 460 706
JP-A-82 203 039
US-A-4 345 079

(73) Proprietor: AIR PRODUCTS AND CHEMICALS, INC.
P.O. Box 538
Allentown, Pennsylvania 18105 (US)

(72) Inventor: Takahashi, Akio
1801 Mill Creek Road
Macungie, PA 18062 (US)
Inventor: Petrella, Robert Gabriel
624 Elm Road
Allentown, PA 18104 (US)
Inventor: Schwartz, Joel
1249 Knossos Drive
Whitehall, PA 18052 (US)

(74) Representative: Kador & Partner
Corneliusstrasse 15
D-8000 München 5 (DE)

Courier Press, Leamington Spa, England.

## Description

Technical field

The invention relates to triethylenediamine crystal and, more particularly, relates to powdery triethylenediamine containing flow control additives.

Background of the invention

Triethylenediamine (TEDA), also known as 1,4-diazabicyclo(2.2.2)octane, is well known in the commercial market as a catalyst or cocatalyst in the production of polyurethane plastics, elastomers and foams. A number of methods are known in the art for preparing and isolating this compound as a product of commercially acceptable purity.

The production of TEDA may employ as a starting material an alkylene polyamine, mono- or bis-hydroxyethyl piperazine, N-aminoethyl piperazine, or alkanolamines alone or in mixture with ethylenediamine.

Typically, the TEDA is isolated from the reaction mixture as a white crystalline hygroscopic product containing a small amount of by-product amine compounds. The TEDA product is generally placed on the market for commercial users in drums of about 25 kg capacity.

With improved purification the synthesized TEDA product is recovered having less than about 500 ppm by-product organic amine impurities. It was found, however, that the purified commercial powdery crystalline TEDA product of this desired low content of organic amine impurities, when stored in commercial size drums for even short periods, particularly in a moderately warm environment, tended to develop a caking, or blocking, problem. This bulk aggregation of the powder crystals is believed the result of two factors, namely (a) sublimation and recrystallization of the TEDA molecules forming a bridge between adjacent particles and (b) hygroscopicity which also results in agglomeration of adjacent particles. Thus the stored powdery product becomes very difficult to remove from the drum by pouring or scooping.

Japanese Patent Publication 82-203039 of Toya Soda Kogyo Co., Ltd. discloses non-solidifying triethylenediamine compositions comprising 0.01—2 parts by weight silica per 100 parts by weight triethylenediamine crystals. The bulk density of the silica should be less than 200 g/l.

U.S. 4,345,079 discloses a scoopable triethylenediamine containing a minor amount of an additive in liquid form which is a polyethylene glycol, a glycol ester, a glycol ether or an amino alcohol. Carbowax 400 polyethylene glycol was the preferred additive.

Summary of the invention

According to the present invention the flowability of triethylene-diamine can be improved when between 0.01 and 1% by weight of certain additives are admixed with the TEDA powder. The materials which are flow improving additives according to the invention are the Group IA, IIA or IIIA metal salts of a $C_8$—$C_{22}$ fatty acid.

TEDA compositions containing additives according to the invention remain as a flowable powder for a longer period and are much easier to render free flowing than TEDA product compositions according to the prior art.

Detailed description of the invention

As flow promoting additives, the fatty acid derivatives may be admixed with the TEDA powder in any manner effective to obtain good dispersion throughout the mass. The admixing may be performed at a temperature ranging from ambient up to the melting point of the TEDA powder. The preferred additives are those which do not adversely affect the appearance or performance of the TEDA or blends of TEDA, such as aqueous solutions or mixtures with dipropylene glycol or dimethylethanolamine in catalyzing urethane and isocyanate formation. The A flow promoting amount of the fatty acid metal salts mixed with the TEDA powder, is 0.01—1 parts of the fatty acid derivative per 100 parts of TEDA. While a degree of improvement in flowability is obtained when about 0.01% of the additive is used, such lower levels have not been found to afford the desired flowability of the TEDA for sufficiently long storage periods at warmer temperatures. At greater than 1 part/100 parts TEDA no additional advantage is obtained.

Although the fatty acid metal salts are relatively water insoluble, this does not, however, present a problem when the TEDA is employed in formulations in which it is dissolved in water, for example in the production of water-blow flexible polyurethane forms, because the actual use level is so low water-solubility may be achieved. Water-solubility is not required where the TEDA is used as catalyst in nonaqueous urethane formulations, isocyanate polymerization or the like provided that the additive is sufficiently soluble in the polyol or other solvent or component of the formulations with which TEDA is to be mixed.

The additives according to the invention are the metal salt of $C_8$—$C_{22}$ fatty acids, preferably fatty acids containing 12—18 carbon atoms. The fatty acids may be saturated in that they comprise long hydrocarbon chains containing no carbon-carbon double bonds or they may be unsaturated fatty acids containing one or more carbon-carbon multiple bonds. Among the fatty acids that are suitable for making the derivatives useful in the invention, there may be included caprylic, capric, myristic, palmitic, stearic, oleic, linoleic, and linolenic acids.

The salt derivatives may be formed by any of the condensation reactions well known in the art such as reacting the fatty acid with the metal hydroxide, carbonate, bicarbonate or alcoholate. Many of the suitable fatty acid salts are commercially available.

The metal salts of the fatty acids comprise as the metal component a Group IA metal such as lithium, sodium, or potassium, a Group IIA metal such as magnesium or calcium; or a Group IIIA metal such as aluminum.

In the following examples the flowability of TEDA was tested and rated as follows:

TEDA containing the indicated amount of a flow promoting additive was mixed in a Hobart mixer at speed #1 (139 gyrations/min) at the specified heating mantle temperature for the specified time.

One pound (0.45 kg) of treated TEDA was placed in a 1 quart (0.95 l) glass jar with a large mouth, capped tightly with a plastic cap and maintained at a selected temperature. Periodically, the jar was uncapped and a screwdriver with a shaft of 1/4"×1/4" (6.4×6.4 mm) thickness and 8" (20.3 cm) length was manually driven through the TEDA in the jar. The flowability was rated by the magnitude of the force required to get the screwdriver through the TEDA mass as follows:

Poor—The screwdriver could not get through the bottom of the jar. The entire TEDA mass was caked and difficult to break up into a powder.

Good—The screwdriver got through to the bottom of the jar. The TEDA mass was caked but could be broken up to a lump and then to a powder with some effort.

Excellent—The screwdriver got through with very little force. The entire TEDA mass broke into free flowing powder.

Example 1

Various additives were subjected to a prescreening test to determine their suitability for improving flowability of plant produced TEDA having a content of by-product amine impurities in the range of about 100—500 ppm. The designated additives were incorporated in the respective amounts set forth in Table I with 900 g of TEDA. After mixing at the indicated temperature for 0.5 hours the product mixture was stored in two jars, one kept at room temperature for 16 hours and the other at 120°F (49°C) for one week.

TABLE I

| Run | Flow additive (g) | TEDA (g) | Mixing treatment (°F) | Flowability 16 hr/RT | Flowability 1 wk./120°F (49°C) |
|---|---|---|---|---|---|
| 1 | | untreated | | poor | poor |
| 2 | | Toya Soda[g] | | very good | poor |
| 3 | Carbowax® 400[a] (9) | 900 | RT | fair | poor |
| 4 | Hi Sil 233[b] (0.9) | 900 | RT | good | poor |
| 5 | Polypropylene Glycol[c] (4.5) | 900 | RT | fair | fair |
| 6 | Ca-Stearate (0.9) | 900 | RT | excellent | excellent |
| 7 | Polyethylene Glycol[d] (4.5) | 900 | 250—300 (121—149°C) | good | poor |
| 8 | Polyethylene glycol monomethyl ether[e] (4.5) | 900 | 250—300 (121—149°C) | good | fair |
| 10 | Na-Stearate (0.9) | 900 | RT | very good/ excellent | excellent |
| 11 | Al-Stearate (0.9) | 900 | RT | very good/ excellent | excellent |

[a] Polyethylene glycol (400 molecular weight) marketed by Union Carbide
[b] A colloidal silica marketed by PPG Industries, Inc.
[c] 4,000 molecular weight
[d] 18,000 molecular weight
[e] 1,900 molecular weight
[g] TEDA marketed by Toya Soda Kogyo Co., Ltd. believed to contain silica

It can be seen from Table I that untreated TEDA powder (Run 1) demonstrated poor flowability after 16 hours at room temperature and one week at 120°F (49°C). The Toya Soda TEDA product which is believed to contain silica as a flow aid (Run 2) showed very good flowability after the 16 hour/room temperature storage but showed poor flowability after one week at 120°F (49°C). Run 4 which used a colloidal silica additive gave similar performance.

Polypropylene glycol (Run 5) and polyethylene glycol (Run 7) present at a level of 4.5 grams per 900 grams TEDA showed fair and good flowability, respectively, at 16 hours/room temperature and were only rated fair and poor, respectively, at one week/120°F (49°C). The monomethyl ether of polyethylene glycol (Run 8) while rated good during the room temperature test deteriorated to a fair rating over the longer test at higher temperature.

The salts of stearic acid, namely the calcium salt (Run 6), the sodium salt (Run 10) and the aluminum salt (Run 11) were all rated very good/excellent when tested after 16 hours/room temperature and, surprisingly, were all rated excellent after a week at the elevated temperature. This is in contrast to the prior art additives (Runs 3—5, 7 and 8) which appeared to lose performance over the extended time period and elevated temperatures.

Example 2

This Example presents flowability results comparing stearate salt additive versus the polyethylene glycol and silica prior art additives. The indicated amount of additive was incorporated for 100 parts powdery TEDA crystal by mixing for 0.5 hr. at room temperature in the Hobart mixer at 139 gyrations per minute. The flow additive treated TEDA was then tested for flowability at three different conditions, namely room temperature for 16—72 hours, 120°F (49°C) for 7 days and 120°F (49°C) for 14 days.

It can be seen from Table II that Runs 16—18 using the prior art treated TEDA all demonstrated significant deterioration in the flowability on exposure to elevated temperatures (120°F, 49°C) for extended periods (1 and 2 weeks). In marked contrast, the sodium, aluminum and calcium salts of stearic acid all showed excellent flowability under the test conditions.

TABLE II

| | | | Flowability | | |
|---|---|---|---|---|---|
| Run | Flow additive[a] (pbw) | Mixing treatment °F | RT/16—72 hrs. | 120°F (49°C) 7 days | 120°F (49°C) 14 days |
| 16 | Carbowax 400 (1.0) | RT | Fair | Poor | Poor |
| 17 | Toyo Soda TEDA (—) | — | Very good | Poor | Very poor |
| 18 | HI SIL 233 (0.1) | RT | Good | Poor | — |
| 19 | Na-Stearate (0.1) | RT | Excellent | Excellent | Excellent |
| 20 | Na-Stearate (0.025) | RT | Excellent | Very good/ excellent | Very good/ excellent |
| 21 | Al-Stearate (0.1) | RT | Excellent | Excellent | — |
| 22 | Ca-Stearate (0.1) | RT | Excellent | Excellent | — |

[a] Part by weight per 100 parts TEDA

Statement of industrial application

The invention provides for improving the flowability of stored powdery TEDA crystals by the incorporation of a flow improving amount of a derivative of a fatty acid.

**Claims**

1. A method for improving the flowability of stored triethylenediamine which comprises mixing with the triethylenediamine between 0.01 and 1% by weight of a flow promoting additive which is a Group IA, IIA or IIIA metal salt of a $C_8$—$C_{22}$ fatty acid.

2. The method of Claim 1 in which the salt is a sodium, calcium or aluminium salt.

3. The method of Claim 1 or 2 in which the fatty acid contains 12 to 18 carbon atoms.

4. The method of Claim 3 in which the fatty acid derivative is sodium stearate.

5. A composition consisting essentially of triethylenediamine admixed with between 0.1 and 1% by weight of a flow promoting additive which is a group IA, IIA or IIIA metal salt of a $C_8$—$C_{22}$ fatty acid.

4

6. The composition of Claim 5 in which the fatty acid salt is a sodium, calcium or aluminium salt.

7. The composition of Claim 5 or 6 in which the fatty acid contains 12 to 18 carbon atoms.

8. The composition of Claim 7 in which the fatty acid derivative is sodium stearate.

**Patentansprüche**

1. Verfahren zur Verbesserung des Fließvermögens von gelagertem Triethylendiamin, bei dem das Triethylendiamin mit zwischen 0,01 und 1 Gew.-% eines das Fließvermögen verbessernden Zusatzstoffes vermischt wird, der ein Metallsaltz der Gruppe IA, IIA oder IIIA einer $C_8$—$C_{22}$-Fettsäure ist.

2. Verfahren nach Anspruch 1, worin das Salz das Natrium-, Calcium- oder Aluminiumsalz ist.

3. Verfahren nach Anspruch 1 oder 2, worin die Fettsäure 12 bis 18 Kohlenstoffatome enthält.

4. Verfahren nach Anspruch 3, worin das Fettsäurederivat Natriumstearate ist.

5. Zusasmmensetzung, die im wesentlichen aus Triethylendiamin besteht, das mit zwischen 0,01 und 1 Gew.-% eines das Fließvermögen verbessernden Zusatzstoffes vermischt ist, der ein Metallsalz der Gruppe IA, IIA oder IIIA einer $C_8$—$C_{22}$-Fettsäure ist.

6. Zusasmmensetzung nach Anspruch 5, worin das Fettsäuresalz das Natrium-, Calcium- oder Aluminiumsalz ist.

7. Zusammensetzung nach Anspruch 5 oder 6, worin die Fettsäure 12 bis 18 Kohlenstoffatome enthält.

8. Zusammensetzung nach Anspruch 7, worin das Fettsäurederivat Natriumstearat ist.

**Revendications**

1. Un procédé pour améliorer la fluidité de la triéthylène-diamine stockée, qui comprend le mélange avec la triéthylènediamine de 0,01 à 1% en poids d'un additif améliorant l'écoulement qui est un sel de métal des groupes IA, IIA ou IIIA d'un acide gras en $C_8$—$C_{22}$.

2. Le procédé de la revendication 1, dans lequel le sel est un sel de sodium, de calcium ou d'aluminium.

3. Le procédé de la revendication 1 ou 2, dans lequel l'acide gras contient 12 à 18 atomes de carbone.

4. Le procédé de la revendication 3, dans lequel le dérivé d'acide gras est le stéarate de sodium.

5. Une composition constituée essentiellement de triéthylènediamine mélangée avec entre 0,01 et 1% en poids d'un additif améliorant l'écoulement qui est un sel de métal des groupes IA, IIA ou IIIA d'un acide gras en $C_8$—$C_{22}$.

6. La composition de la revendication 5, dans laquelle le sel d'acide gras est un sel de sodium, de calcium ou d'aluminium.

7. La composition de la revendication 5 ou 6, dans laquelle l'acide gras contient 12 à 18 atomes de carbone.

8. La composition de la revendication 7, dans lequelle le dérivé d'acide gras est le stéarate de sodium.